# FASCICULE DE BREVET EUROPEEN

(11) **EP 3 577 195 B1**
(45) Date de publication et mention de la délivrance du brevet: **23.06.2021**
(21) Numéro de dépôt: 17828948.4
(22) Date de dépôt: 15.12.2017
(51) Int. Cl.: C09K 8/528, G01N 21/64

(54) **METHODE DE DETERMINATION DE LA DENSITE DE CHARGE ANIONIQUE D'UN POLYMERE**
VERFAHREN ZUR BESTIMMUNG DER ANIONISCHEN LADUNGSDICHTE EINES POLYMERS
METHOD OF DETERMINING THE ANIONIC CHARGE DENSITY OF A POLYMER

(30) Priorité: 31.01.2017 FR 1750814
(43) Date de publication de la demande: 11.12.2019
(73) Titulaire: SPCM SA, 42160 Andrézieux-Bouthéon (FR)
(72) Inventeur: FAVERO, Cédrick, 42160 Andrézieux Bouthéon (FR); BRAUN, Olivier, 42160 Andrézieux Bouthéon (FR); SOUZY, Renaud, 42160 Andrézieux Bouthéon (FR); MARAIS, Arthur, 56960 Lochrist (FR); BRICHART, Thomas, 69006 Lyon (FR)
(74) Mandataire: Cabinet Laurent & Charras
(86) Numéro de dépôt international: PCT/FR2017/053624
(87) Numéro de publication internationale: WO 2018/142034

(56) Documents cités:
- WO-A1-2004/021004
- WO-A1-2015/075299
- WO-A1-2016/203119
- Eva F Gudgin Dickson ET AL: "Ultrasensitive bioanalytical assays using time-resolved fluorescence detection", Pharmacology and Therapeutics, 1 janvier 1995 (1995-01-01), pages 207-235, XP055407147, ENGLAND DOI: 10.1016/0163-7258(94)00078-H Extrait de l'Internet: URL:http://ac.els-cdn.com/016372589400078H /1-s2.0-016372589400078H-main.pdf?_tid=887 8b44a-9a0e-11e7-b4b5-00000aab0f6c&acdnat=1 505477466_a1240e3ef3043bd5efcb40f52505fd53 [extrait le 2017-10-09]

## Description

### DOMAINE DE L'INVENTION

La présente invention concerne une méthode de détermination de la densité de charge anionique de polymère(s). Plus précisément, la présente invention concerne une méthode de détermination de la densité de charge anionique de polymère(s) présent(s) dans un échantillon en utilisant la photoluminescence résolue en temps.

### ETAT ANTERIEUR DE LA TECHNIQUE

Les polymères anioniques sont utilisés dans des domaines variés tel que le traitement de l'eau, le traitement des boues, ou la récupération assistée du pétrole et du gaz. Dans ces différents domaines, il est important de connaitre les caractéristiques des polymères et notamment leur densité de charge anionique. Par exemple, dans le domaine de la récupération assistée du pétrole, les polymères subissent des dégradations sous l'effet de la pression, de la température et de l'environnement chimique au sein de la formation souterraine. A la sortie du puits, le polymère a une densité de charge anionique inconnue du fait de ces dégradations.

De nos jours, différentes techniques analytiques sont utilisées pour déterminer la densité de charge anionique des polymères. Comme exemples de techniques, nous pouvons citer la titration par conductimétrie, la teneur en azote, la titration par potentiométrie, la spectroscopie RMN ¹³C, la spectroscopie infrarouge, la spectroscopie UV, l'analyse thermogravimétrique ou encore la calorimétrie.

Toutefois, ces méthodes sont très contraignantes car, pour la plupart, il est indispensable d'avoir un échantillon relativement pur, ce qui nécessite des étapes de purification complexes. De plus, avec ces méthodes, il est indispensable de connaître la concentration en polymère de l'échantillon, caractéristique qui n'est pas toujours connue, difficilement mesurable et souvent dépendante de la densité de charge anionique.

Il existe donc un besoin concernant une méthode de détermination de la densité de charge anionique des polymères dans un échantillon sans connaitre la concentration en polymère(s) mais qui reste simple et pratique.

### EXPOSE DE L'INVENTION

La présente invention a pour objet une méthode de détermination de la densité de charge anionique d'au moins un polymère présent dans un échantillon. Cette méthode comprend les étapes suivantes :
- optionnellement, prétraiter l'échantillon,
- mettre en contact et permettre l'interaction du au moins un polymère présent dans l'échantillon avec une solution révélatrice comprenant des ions lanthanides (III),
- exciter l'échantillon à une longueur d'onde d'excitation λ_{exc} et détecter, par photoluminescence résolue en temps, un signal provenant des ions lanthanides (III) ayant interagit avec le au moins un polymère à une longueur d'onde d'émission λₑₘ, et
- déterminer la densité de charge anionique du au moins un polymère de l'échantillon en utilisant le signal détecté à la longueur d'onde d'émission λₑₘ, selon le protocole suivant :
   1. on prépare différentes séries d'échantillon par dilution successive de différentes solutions mères de polymère d'anionicité connue avec de l'eau, les échantillons de chacune des séries, sont ensuite dilués avec une solution révélatrice de lanthanide et analysés par Fluorescence en Temps Résolu (FTR) ; les paramètres de mesure ainsi que les longueurs d'onde d'émission, d'excitation étant réglés en fonction de la nature du lanthanide,
   2. pour chacune des séries, les pentes des courbes Intensités Signal FTR vs Taux de Dilution sont extrapolées et une courbe de calibration Pente vs Anionicité est élaborée,
   3. on prépare ensuite par dilution successive d'un échantillon X d'anionicité inconnue, une nouvelle série d'échantillons, après mesure FTR de ces échantillons, la pente de la courbe Intensité Signal FTR vs Taux de Dilution est extrapolée,
   4. après corrélation de la pente extrapolée en 3) avec la droite de calibration d'anionicité élaborée en 2), l'anionicité préalablement inconnue de l'échantillon X est déduite.

Les étapes de dilution peuvent être réalisées par addition d'eau.

La méthode de la présente invention peut être utilisée pour déterminer la densité de charge anionique de polymères présents dans des échantillons provenant de formations souterraines comme les puits de pétrole ou de gaz ; des procédés de traitement de l'eau ou de boue ; de la cosmétique ; de la détergence ; de la fabrication du papier ; et ou l'industrie minière. De manière préférentielle, la méthode de la présente invention est utilisée pour déterminer la densité de charge anionique de polymères présents dans des échantillons provenant de formations souterraines, notamment les puits de pétrole ou de gaz. Les échantillons proviennent avantageusement d'un puits de production, plus particulièrement d'une eau de production issue d'un procédé de récupération du pétrole ou du gaz. La détermination de la densité de charge anionique permet d'obtenir des informations sur les dégradations que subissent les polymères au sein de la formation souterraine. De plus, dans le cas de la réinjection de l'eau de production, il est important de connaitre la densité charge anionique afin de réajuster de manière optimale la densité de charge anionique de la solution.

Il a été découvert de manière totalement surprenante qu'en utilisant la méthode selon l'invention, le signal obtenu par photoluminescence résolue en temps, du produit de l'interaction entre les polymères et la solution révélatrice comprenant les ions lanthanides, est en corrélation précise avec la densité de charge anionique des polymères présents dans un échantillon.

Cette méthode a l'avantage d'être totalement indépendante du poids moléculaire des polymères. Ainsi, cette méthode convient parfaitement pour les polymères ayant un poids moléculaire compris entre 1000 g/mol et 35 millions g/mol. Sauf indication contraire, par « poids moléculaire » d'un polymère, on entend le poids moléculaire moyen en masse.

Selon l'invention, le polymère peut être un polymère naturel pouvant être choisi parmi les polysaccharides tel que l'amidon, la guar, la cellulose, le dextran ou le xanthane. Selon l'invention le polymère peut aussi être un polycondensat. De manière avantageuse, l'échantillon comprend au moins un polymère ayant une ou plusieurs charge(s) anionique(s). De manière avantageuse, tous les polymères présents dans l'échantillon comprennent une ou plusieurs charge(s) anionique(s).

Le polymère peut être un copolymère formé d'au moins deux ou plusieurs monomères.

Selon l'invention, le polymère peut avoir une structure linéaire, branchée, réticulée, star (en forme d'étoile) ou comb (en forme de peigne).

Selon l'invention, le polymère peut être obtenu par copolymérisation d'au moins un monomère anionique et d'au moins un monomère (A) non ionique et optionnellement d'au moins un monomère cationique ou zwitterionique.

Le ou les monomères anioniques peuvent être choisis dans un large groupe. Il s'agit avantageusement d'un monomère hydrosoluble, c'est-à-dire d'un monomère soluble dans l'eau dans les conditions conventionnelles de polymérisation. Ces monomères peuvent présenter des fonctions acryliques, vinyliques, maléiques, fumariques, malonique, itaconique, allyliques. Ils peuvent contenir un groupe carboxylate, phosphonate, phosphate, sulfate, sulfonate, ou un autre groupe à charge anionique. Le monomère anionique peut être sous forme acide ou bien sous forme de sel de métal alcalino-terreux ou de métal alcalin. Des exemples de monomères convenables comprennent l'acide acrylique ; l'acide méthacrylique ; l'acide itaconique ; l'acide crotonique ; l'acide maléique ; l'acide fumarique ; les monomères de type acide fort présentant par exemple une fonction de type acide sulfonique ou acide phosphonique tels que l'acide 2-acrylamido 2-methylpropane sulfonique, l'acide vinylsulfonique, l'acide vinylphosphonique, l'acide allylsulfonique, l'acide allylphosphonique, ou l'acide styrène sulfonique ; et les sels hydrosolubles de ces monomères comme leurs sels de métaux alcalins, de métaux alcalino-terreux, ou d'ammonium.

Selon un mode de réalisation particulier, le polymère comprend avantageusement entre 1 et 99 mol% de monomère(s) anionique(s), de préférence entre 3 et 80 mol%, et encore plus préférentiellement entre 5 et 50 mol%, par rapport au nombre total de moles de monomères.

Le monomère (A) peut être un monomère non-ionique pouvant notamment être choisi dans le groupe comprenant les monomères vinyliques hydrosolubles, et particulièrement l'acrylamide ; le méthacrylamide, le N-isopropylacrylamide ; le N,N-diméthylacrylamide ; la N-vinylformamide ; l'acryloyl morpholine ; le N,N-diéthyle acrylamide ; le N-tert butyl acrylamide ; le N-tert-octylacrylamide, ; la N-vinylpyrrolidone ; la N-vinyl caprolactame ; la N-vinyl imidazole ; et la diacetone acrylamide.

Le monomère non ionique peut aussi être choisi parmi les monomères de formule : D-Z-D'
où :
- D est une fonction chimique insaturée polymérisable du type acrylate, méthacrylate, acrylamido, méthacrylamido, vinylique ou allylique,
- D' représente l'hydrogène ou un groupe alkyl (avantageusement en C₁-C₂₂) ou aryl (avantageusement en C₁-C₂₂),
- Z a la structure suivante : -(OE)w-(OP)x-(OBu)z- où :
   - OE, OP, OBu désignent respectivement l'oxyde d'éthylène (-CH₂-CH₂-O-), l'oxyde de propylène (-CH₂-CH₂-CH₂-O-) et l'oxyde de butylène (-CH₂-CH₂-CH₂-CH₂-O-).
   - L'arrangement entre les différents motifs d'OE et/ou d'OP et/ou d'OBu peut être statistique, alterné, gradient ou bloc.
   - w, x et z sont des entiers compris entre 0 et 150 et w + x + z ≠ 0.

Selon un mode de réalisation particulier, le polymère comprend avantageusement entre 1 et 99,9 mol% de monomère(s) non-ionique(s), de préférence entre 40 et 95 mol% et plus préférentiellement entre 60 et 90 mol%, par rapport au nombre total de moles de monomères.

Le monomère cationique peut être de type acrylamide, acrylique, vinylique, allylique ou maléique et posséder une fonction amine ou ammonium quaternaire. On peut citer, en particulier et de façon non limitative, l'acrylate de diméthylaminoéthyl (ADAME), et le méthacrylate de diméthylaminoéthyle (MADAME) quaternisés ou salifiés, le chlorure de diméthyldiallylammonium (DADMAC), le chlorure d'acrylamido propyltriméthyl ammonium (APTAC) et le chlorure de méthacrylamido propyltriméthyl ammonium (MAPTAC). Les monomères cationiques dérivés de l'acrylamide et portant une chaine hydrophobe décrits dans le document FR 2 868 783 peuvent être utilisés.

Selon un mode de réalisation particulier, le polymère comprend avantageusement entre 1 et 30 mol% de monomère(s) cationique(s), de préférence entre 2 et 20 mol% et plus préférentiellement entre 5 et 15 mol%, par rapport au nombre total de moles de monomères.

Selon l'invention, la solution révélatrice comprend des ions lanthanides (III). Les ions lanthanides (III) sont avantageusement choisis parmi les ions europium, terbium, samarium ou dysprosium. Préférentiellement les ions lanthanide (III) sont des ions europium ou des ions terbium. Dans la solution révélatrice, le lanthanide (III) peut être un sel de lanthanide comme par exemple un halogénure de lanthanide tel que le chlorure d'europium.

Selon un mode particulier de l'invention, la solution révélatrice peut comprendre une ou plusieurs solutions tampons afin d'améliorer le rapport signal sur bruit des échantillons analysés. Parmi les tampons pouvant être utilisés, nous pouvons citer à titre d'exemple, les dérivés d'acide sulfonique, tels que, par exemple HEPES (acide 2- [4- (2-hydroxyéthyl) pipérazin-1 -yl] éthanesulfonique, pKa 7,48), PIPES (1, 4- acide piperazinediethanesulfonic, pKa 6,76), MOPS (3-morpholinopropane-1 - sulfonique, pKa 7,2) et MES (2 - (N-morpholino) éthanesulfonique, pKa 6,15). Préférentiellement le tampon est l'HEPES. Des solutions révélatrices pouvant être utilisées sont notamment commercialisées par la société Glincs.

La solution révélatrice est avantageusement une solution aqueuse.

Selon un autre mode particulier de l'invention, un ou plusieurs tampons, cités ci-dessus, peuvent être ajoutés à l'échantillon avant la détection du signal à la longueur d'onde d'émission λₑₘ, afin d'améliorer le rapport signal sur bruit et le rapport signal sur bruit de fond des signaux des échantillons détectés.

La quantité d'ions lanthanide (III) introduits dans l'échantillon est avantageusement comprise entre 1 ppm et 10000 ppm, préférentiellement entre 5 ppm et 1000 ppm. La quantité d'ions lanthanide (III) est exprimée en poids par rapport au poids de l'échantillon avant la mise en contact de l'échantillon avec la solution révélatrice.

Selon l'invention, la densité de charges anioniques est quantifiée, en utilisant une méthode de photoluminescence résolue en temps qui est notamment décrite dans l'article *"*Ultrasensitive bioanalytical assays using time resolved fluorescence detection", Pharmacol. Ther., Vol. 66(2), pages 207-35, 1995. Celle-ci repose sur l'application d'un délai, dit délai d'intégration, entre l'excitation de l'échantillon à analyser et la mesure du signal émis, de manière à s'affranchir des photoluminescences parasites à durée de vie courte. Cette méthode peut être mise en œuvre à température ambiante, notamment à l'aide d'un appareil de type Cary Eclipse de la société Agilent.

La longueur d'onde, qui est utilisée dans l'invention, peut être sélectionnée ou déterminée en étudiant l'excitation maximum dans le spectre d'excitation du produit de l'interaction entre les polymères et la solution révélatrice comprenant des ions lanthanides (III). Par exemple la longueur d'onde d'excitation λ_{exc} peut être comprise entre 200 nm et 600 nm et la longueur d'onde λₑₘ du signal d'émission peut être comprise entre 300 nm et 800 nm.

Le délai d'intégration peut être compris entre 0,001 ms et 10 ms (ms = millisecondes), de préférence entre 0,01 et 5 ms, plus préférentiellement entre 0,1 et 3 ms. Dans certains cas, plus ce délai est long, meilleur est le rapport signal / bruit, ce qui améliore la fiabilité de la mesure. La durée de récolte des photons peut aller de 5 à 10 ms, par exemple.

Selon un mode de réalisation de l'invention, un modificateur de signal comprenant un composé cationique, peut être ajouté à l'échantillon avant l'excitation de l'échantillon. Le modificateur de signal peut être utilisé pour modifier le signal d'échantillonnage, par exemple son intensité, ou pour modifier la différence entre les longueurs d'onde d'excitation pour les différents polymères. Le modificateur de signal peut comprendre un ion métallique qui est avantageusement choisi parmi le cuivre, le nickel, le chrome, le fer, l'or, l'argent, le cobalt, et leurs mélanges. De préférence, le modificateur de signal comprend du cuivre (II). Le modificateur de signal peut comprendre un polymère cationique de bas poids moléculaire, avantageusement inférieur à 25000 g/mol. L'échantillon peut de manière optionnelle être prétraité avant la détermination de la densité de charge anionique. Ce prétraitement peut s'avérer utile lorsque l'échantillon comprend des sels, par exemple des sels inorganiques présents dans l'eau de production, ou des particules insolubles. L'eau de production correspond à l'eau récupérée après la séparation eau/hydrocarbures dans un procédé de récupération du pétrole ou du gaz.

Selon un mode de réalisation de l'invention, l'échantillon peut être purifié avant l'addition de la solution révélatrice comprenant les ions lanthanides (III) afin d'éliminer des substances et/ou des composés interférant le signal mesuré à la longueur d'onde d'émission λₑₘ. Par exemple, un pré-nettoyage peut aider à minimiser le bruit de fond provoqué par les composants de l'échantillon. Parmi les procédés de purification pouvant être utilisés dans le cadre de l'invention, nous pouvons citer à titre d'exemple, la centrifugation, la chromatographie d'exclusion de taille, le nettoyage avec des cartouches d'extraction en phase solide (SPE), les techniques de dialyse, les méthodes d'extraction pour l'élimination des hydrocarbures, la filtration, la microfiltration, l'ultrafiltration, la nanofiltration, la centrifugation sur membrane et / ou d'autres méthodes permettant de séparer les espèces polymériques ayant un petit poids moléculaire (avantageusement inférieur à 1000 g/mol).

Selon un mode de réalisation de l'invention, la concentration en sel de l'échantillon peut être modifiée et/ou les particules insolubles peuvent être éliminées avant l'addition de la solution révélatrice comprenant les ions lanthanides (III). La modification de la concentration en sel de l'échantillon peut se traduire par une diminution ou une augmentation de la concentration en sel avant l'addition de la solution révélatrice comprenant les ions lanthanides (III).

Selon un mode particulier de l'invention, si l'échantillon est trop visqueux à cause d'une concentration initiale en polymères trop importante, l'échantillon peut être dilué avant l'addition de la solution révélatrice comprenant les ions lanthanides (III). Les diluants peuvent être choisis parmi l'eau, des solutions tampons aqueuses, des solutions salines saturées ou non en sels, ou leurs mélanges. Comme déjà indiqué, il n'est pas nécessaire de connaitre la concentration de l'échantillon en polymère afin de déterminer sa densité de charge anionique.

Selon un mode particulier de l'invention, une ou plusieurs des étapes de prétraitement ci-dessus peuvent être effectuées sur un échantillon avant la mesure de la densité de charge anionique d'un échantillon. Par exemple, avant la mesure, l'échantillon peut être purifié et/ou dilué.

Selon un mode particulier de l'invention, la valeur du pH de l'échantillon est ajustée à un niveau approprié. Le pH de l'échantillon est avantageusement compris entre 3 et 8, de préférence entre 5 et 8. Tout tampon approprié qui ne perturbe pas de manière significative la détection du signal échantillon, peut être utilisé. Des exemples de tampons sont donnés ci-dessus, mais d'autres tampons peuvent également être utilisés. Cependant, lorsque l'échantillon provient d'une eau de production, toutes les étapes de dilution peuvent être réalisées avec une saumure ayant les mêmes caractéristiques de conductivité et de salinité que l'eau de production, même pour les polymères standards.

Comme déjà indiqué, il n'est pas nécessaire de connaître la concentration en polymère, même en polymère standard pour mettre en œuvre l'invention.

L'invention et les avantages qui en découlent ressortiront mieux des figures et des exemples suivants donnés afin d'illustrer l'invention et non de manière limitative.

### DESCRIPTION DES FIGURES

La figure 1 illustre le graphe de l'intensité du signal à la longueur d'onde d'émission λₑₘ en fonction de la dilution d'une solution mère d'un polymère standard.
La figure 2 illustre le graphe de l'intensité du signal à la longueur d'onde d'émission λₑₘ en fonction de la dilution d'une solution mère d'un polymère ayant une densité de charge anionique connue.
La figure 3 illustre le graphe de l'intensité du signal à la longueur d'onde d'émission λₑₘ en fonction de la dilution d'une solution mère d'un polymère ayant une densité de charge anionique connue.
La figure 4 illustre le graphe de l'intensité du signal à la longueur d'onde d'émission λₑₘ en fonction de la dilution d'une solution mère d'un polymère ayant une densité de charge anionique connue.
La figure 5 illustre le graphe de l'intensité du signal à la longueur d'onde d'émission λₑₘ en fonction de la dilution d'une solution mère d'un polymère ayant une densité de charge anionique connue.
La figure 6 illustre la variation linéaire de la pente de chacune des figures 1 à 5.
La figure 7 illustre le graphe de l'intensité du signal à la longueur d'onde d'émission λₑₘ en fonction de la dilution d'une solution mère d'un polymère ayant une densité de charge anionique inconnue.
La figure 8 représente l'intensité du signal en fonction de la longueur d'onde d'émission pour des polymères ayant une densité de charge anionique connue.
La figure 9 représente la pente (intensité en fonction de la dilution) en fonction de l'anionicité pour des polymères ayant une densité de charge anionique connue.
La figure 10 illustre les variations de la pente en fonction de l'anionicité à différents taux de dilution.

### EXEMPLES DE REALISATION DE L'INVENTION

Les abréviations suivantes sont utilisées :
AANa : Acrylate de Sodium
AM : Acrylamide
λₑₘ : Longueur d'onde d'émission
λ_{exc} : Longueur d'onde d'excitation

### Exemple 1 - Dosage de l'anionicité d'un polymère présent dans une eau industrielle

Cet exemple porte sur le dosage de l'anionicité d'un polymère acrylamide/acrylate de sodium présent dans une eau de production issue de la production du pétrole à partir de réservoirs exploités selon les techniques de récupération assistée du pétrole par injection d'une solution à base de polymère.

### a) Préparation des solutions témoins

On prépare les solutions suivantes de différentes concentrations non connues par dilution successive d'une solution mère de polymère avec de l'eau ayant la même salinité que l'eau de production à analyser (Tableau 1).

La salinité de l'eau de production est :
Concentration en NaCl : 25,00 g.L⁻¹
Concentration en CaCl₂ : 1 g.L⁻¹

Une saumure est préparée de manière à obtenir les mêmes caractéristiques de salinité que celles de l'eau de production. Cette saumure est utilisée pour les dilutions des différentes solutions.

### b) Interaction avec des solutions révélatrices

Chacune des solutions listées dans le tableau 1 sont mélangées avec une solution révélatrice de Chlorure de Terbium III commercialisée par la société Glincs, suivant le ratio (1vol /10 vol). Le pH est de 6,5.

**Tableau 1 : Liste des dilutions des différentes solutions mères de polymère.**

| **Solution** | **Polymère** | | | **Dilution dans l'eau de production** |
|---|---|---|---|---|
| | **Référence** | **Composition chimique (mol %)** | | |
| | | **AANa** | **AM** | |
| A-1 | A | 20 | 80 | 1/10 |
| A-2 | | | | 1/20 |
| A-3 | | | | 1/50 |
| A-4 | | | | 1/100 |
| A-5 | | | | 1/150 |
| B-1 | B | 30 | 70 | 1/10 |
| B-2 | | | | 1/20 |
| B-3 | | | | 1/50 |
| B-4 | | | | 1/100 |
| B-5 | | | | 1/150 |
| C-1 | C | 40 | 60 | 1/20 |
| C-2 | | | | 1/50 |
| C-3 | | | | 1/100 |
| C-4 | | | | 1/150 |
| D-1 | D | 50 | 50 | 1/20 |
| D-2 | | | | 1/50 |
| D-3 | | | | 1/100 |
| D-4 | | | | 1/150 |
| E-1 | E | 70 | 30 | 1/10 |
| E-2 | | | | 1/20 |
| E-3 | | | | 1/50 |
| E-4 | | | | 1/100 |
| E-5 | | | | 1/150 |

### c) Mesure de Photoluminescence Résolue en Temps

Les mesures sont effectuées à 20°C dans une cuvette en quartz sur un spectromètre du type Cary Eclipse Fluorescence Spectrophotometer de la société Agilent ayant pour caractéristiques :
- Lampe flash au Xenon de 75 kW
- Monochromateurs Czerny-Turner
- Détecteur PM à 800 V
- Δₚᵤₗₛₑ = 2 µs

Les spectres d'excitation des échantillons sont réalisés entre 200 et 450 nm et la longueur d'onde d'émission λₑₘ est de 545 nm.

Les paramètres de mesure sont réglés de la façon suivante :
- Délai : 0,1 ms
- Durée de récolte photonique : 5 ms
- Fréquence de la lampe : 100 Hz
- Nombre de flash : 1

L'analyse est lancée à l'aide du logiciel contrôlant le spectrofluorimètre.

### d) Dosage de l'anionicité des échantillons témoins

Les spectres d'excitation des différentes solutions du Tableau 1 sont réalisés à λ_{exc} = 320 nm et λₑₘ = 545 nm.

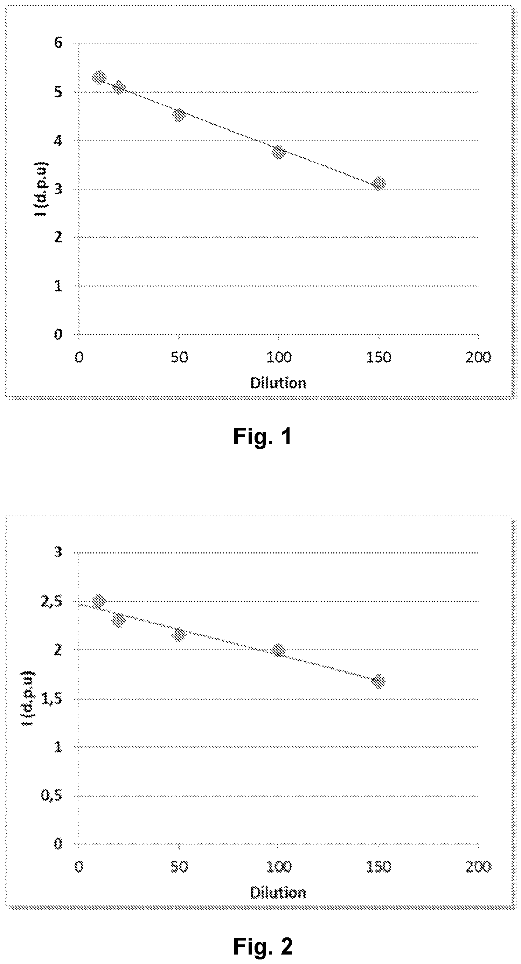

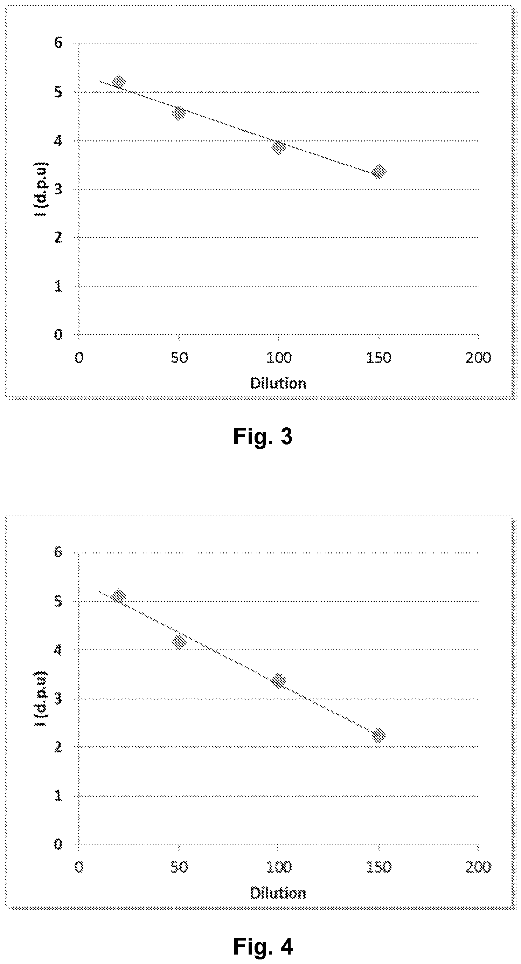

Les intensités des pics en fonction de la dilution sont présentées dans les figures 1 à 5.

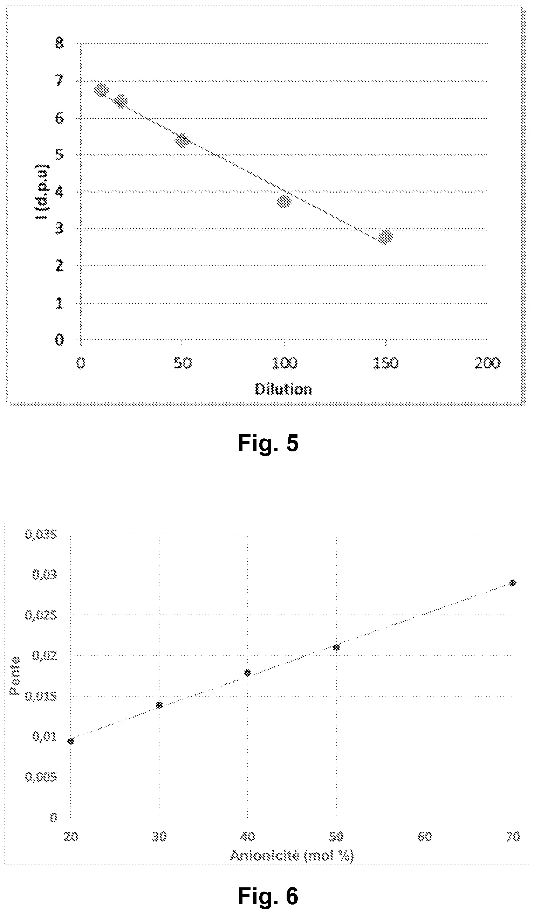

La Figure 6 met en évidence la variation linéaire de la pente de chacune des courbes des figures 1 à 5

### e) Dosage de l'anionicité d'un échantillon X de polymère dilué dans la saumure

Un échantillon X d'anionicité inconnue a été prélevé dans un volume d'eau de production issue de la production du pétrole à partir de réservoirs exploités selon les techniques de récupération assistée du pétrole par injection d'une solution à base de polymère.

On prépare 5 solutions de différentes concentrations non connues par dilution successive de l'échantillon avec de la saumure (voir le paragraphe a) : 25,00 g.L⁻¹ NaCl et 1 g.L⁻¹ CaCl₂).

Chacune de ces 5 solutions sont mélangées (1vol/10vol) avec une solution révélatrice de Chlorure de Terbium III commercialisée par la société Glincs. Le pH est de 6,5.

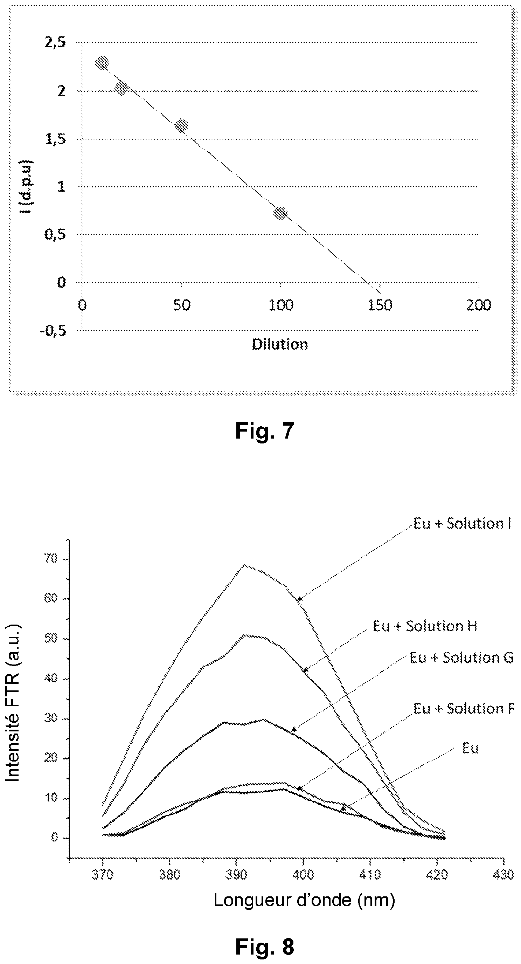

Les intensités obtenues en fonction de la dilution sont rassemblées dans la Figure 7.

Après extrapolation de la pente de la droite de la Figure 7 et corrélation avec la droite de calibration de la Figure 6, on déduit que l'anionicité du polymère est de 39,25 mol%.

### Exemple 2 - Dosage de l'anionicité d'un polymère présent dans un fluide de forage

Cet exemple porte sur le dosage de l'anionicité d'un polymère anionique de composition inconnue présent dans un fluide de forage collecté en sortie de puits de pétrole producteur.

### a) Appareillage & Mesures

Les mesures de photoluminescence Résolue en Temps sont effectuées à température ambiante dans une cuvette en quartz avec un spectromètre du type Cary Eclipse Fluorescence Spectrophotometer de la société Agilent. Les caractéristiques sont identiques à celles de l'Exemple 1.

Les spectres d'excitation des échantillons sont réalisés entre 200 et 450 nm et la longueur d'onde d'émission λₑₘ est de 617 nm.

Les paramètres de mesure sont réglés de la façon suivante :
- Délai : 0,5 ms
- Durée de récolte photonique : 2 ms
- Fréquence de la lampe : 100 Hz
- Nombre de flash : 1

L'analyse est lancée à l'aide du logiciel contrôlant le spectrofluorimètre.

### b) Préparation des échantillons témoins & Spectres d'Excitation

On prépare les solutions suivantes de différentes concentrations non connues par dilution successive d'une solution mère de polymère avec de l'eau ayant la même salinité que le fluide de forage à analyser (Tableau 2).

**Tableau 2 : Anionicité des références témoins F à I**

| Polymère | |
|---|---|
| Référence | Anionicité (mol%) |
| F | 15 |
| G | 25 |
| H | 35 |
| I | 45 |

Chacune des solutions listées dans le tableau 2 sont mélangées (1vol/10vol) avec une solution révélatrice de Chlorure d'Europium III commercialisée par la société Glincs. Le pH est de 6,5.

La Figure 8 montre une modification de l'intensité des pics sur le spectre d'excitation traduisant une complexation des ions Europium avec les solutions de l'étude.

### c) Dosage de l'anionicité des échantillons témoins

Les spectres d'excitation des différentes solutions du Tableau 2 sont réalisés à λ_{exc} = 395 nm et λₑₘ = 617 nm.

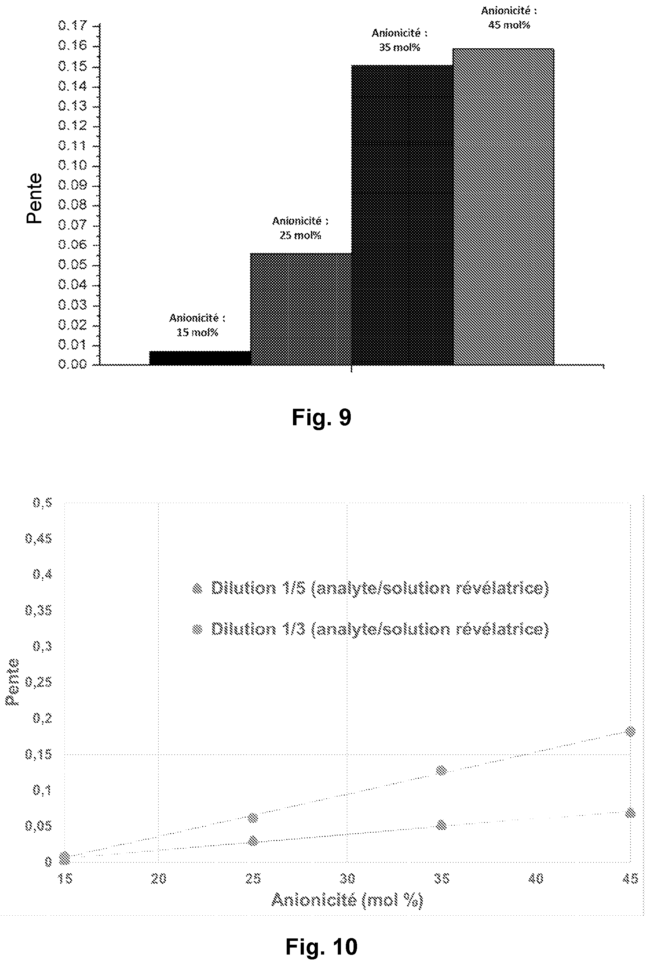

La Figure 9 représente les pentes obtenues en fonction des anionicités des témoins.

### d) Dosage de l'anionicité inconnue de l'échantillon du fluide forage

Un échantillon d'anionicité inconnue a été prélevé dans le fluide de forage. Il a été filtré afin d'éliminer les insolubles. On prépare des solutions de différentes concentrations (non connue) par dilution successive de l'échantillon avec de l'eau ayant la même salinité que le fluide de forage à analyser. Les 5 solutions sont mélangées (1vol/10vol) avec une solution révélatrice de Chlorure d'europium III commercialisée par la société Glincs. Le pH est de 6,5.

La pente issue de la variation linéaire de l'intensité des pics de fluorescence obtenus en fonction de la dilution est de 0,152. Après corrélation avec la droite de calibration de la Figure 9, on déduit que l'anionicité du polymère est de 36,17 mol%.

### Exemple 3 - Amélioration de la sensibilité du dosage de l'anionicité d'un copolymère

Cet exemple porte sur l'utilisation de conditions opératoires permettant d'améliorer la sensibilité et la résolution de la mesure du dosage de l'anionicité d'un copolymère acrylamide / acrylate de sodium présent, par exemple, dans une eau de production du pétrole à partir de réservoirs exploités selon les techniques de récupération assistée du pétrole à base de polymère ou dans un fluide de forage.

### a) Mesure de Luminescence en Temps Résolu

Les mesures sont effectuées à 20°C dans une cuvette en quartz sur un spectromètre du type Cary Eclipse Fluorescence Spectrophotometer de la société Agilent ayant pour caractéristiques :
- Lampe flash au Xenon de 75 kW
- Monochromateurs Czerny-Turner
- Détecteur PM à 800 V
- Δpulse = 2 µs

Les spectres d'excitation des échantillons sont réalisés entre 200 et 450 nm. La longueur d'onde d'émission est λₑₘ = 617 nm.

Les paramètres de mesure sont réglés de la façon suivante :
- Délai : 0,5 ms
- Durée de récolte photonique : 2 ms
- Fréquence de la lampe : 100 Hz
- Nombre de flash : 1

L'analyse est lancée à l'aide du logiciel contrôlant le spectrofluorimètre.

### b) Préparation des solutions témoins et dosage de l'anionicité

On prépare des solutions de différentes concentrations non connues par dilution successive d'une solution mère de polymère avec une saumure ([NaCl] = 150 g/L) (tableau 3).

Les spectres d'excitation de ces différentes solutions sont réalisés à λ_{exc} = 395 nm et λₑₘ = 617 nm. Chacune des solutions listées dans le tableau 1 sont mélangées avec une solution révélatrice d'Europium III commercialisée par la société Glincs. Le pH est de 6,5.

**Tableau 3 : Anionicité des polymères témoins R1 à R4**

| **Polymère** | |
|---|---|
| **Référence** | **Anionicité (mol%)** |
| R1 | 15 |
| R2 | 25 |
| R3 | 35 |
| R4 | 45 |

Les variations de la pente en fonction de l'anionicité à différents taux de dilution (Analyte) / (Solution Révélatrice) ont été obtenues (figure 10).

Un échantillon d'anionicité inconnue a été prélevé dans un fluide de forage. Il a été filtré afin d'éliminer les insolubles. On prépare 5 solutions de différentes concentrations (non connues) par dilution successive de l'échantillon avec de la saumure. Chacune de ces 5 solutions sont mélangées (1vol/3vol) avec une solution révélatrice d'europium III commercialisée par la société Glincs. Le pH est de 6,5. La pente issue de la variation linéaire de l'intensité des pics de fluorescence obtenue en fonction de la dilution est de 0,095. Après corrélation avec la droite de calibration de la figure 10, on déduit que l'anionicité du polymère est de 30 mol%.

Cet exemple démontre une nouvelle fois qu'il est possible de doser l'anionicité d'un polymère par la technique de fluorescence en temps résolu et qu'il est possible d'améliorer la sensibilité de la mesure en travaillant en milieu plus concentré en sel.

## Revendications

1. Méthode de détermination de la densité de charge anionique d'au moins un polymère présent dans un échantillon, selon les étapes suivantes :
- mettre en contact et permettre l'interaction du au moins un polymère présent dans l'échantillon avec une solution révélatrice comprenant des ions lanthanides (III),
- exciter l'échantillon à une longueur d'onde d'excitation λ_{exc} et détecter, par photoluminescence résolue en temps, un signal provenant des ions lanthanides (III) ayant interagit avec le au moins un polymère à une longueur d'onde d'émission λₑₘ, et
- déterminer la densité de charge anionique du au moins un polymère de l'échantillon en utilisant le signal détecté à la longueur d'onde d'émission λₑₘ selon le protocole suivant :
1) on prépare différentes séries d'échantillon par dilution successive de différentes solutions mères de polymère d'anionicité connue avec de l'eau, les échantillons de chacune des séries, sont ensuite dilués avec une solution révélatrice de lanthanide et analysés par Fluorescence en Temps Résolu (FTR) ; les paramètres de mesure ainsi que les longueurs d'onde d'émission, d'excitation étant réglés en fonction de la nature du lanthanide,
2) pour chacune des séries, les pentes des courbes Intensités Signal FTR vs Taux de Dilution sont extrapolées et une courbe de calibration Pente vs Anionicité est élaborée,
3) On prépare ensuite par dilution successive d'un échantillon X d'anionicité inconnue, une nouvelle série d'échantillons,
après mesure FTR de ces échantillons, la pente de la courbe Intensité Signal FTR vs Taux de Dilution est extrapolée,
4) après corrélation de la pente extrapolée en 3) avec la droite de calibration d'anionicité élaborée en 2), l'anionicité préalablement inconnue de l'échantillon X est déduite.

2. Méthode selon la revendication 1, ***caractérisée* en ce que** l'échantillon provient d'une eau de production issue d'un procédé de récupération du pétrole ou du gaz.

3. Méthode selon l'une des revendications précédentes, ***caractérisée* en ce que** le au moins un polymère présent dans l'échantillon est un polymère d'au moins un monomère hydrosoluble anionique et d'au moins un monomère (A) non ionique et, optionnellement, d'au moins un monomère cationique ou zwitterionique.

4. Méthode selon l'une des revendications précédentes, ***caractérisée* en ce que** les ions lanthanides sont choisis parmi les ions europium, terbium, samarium et dysprosium.

5. Méthode selon l'une des revendications précédentes, ***caractérisée* en ce que** les ions lanthanides sont les ions europium ou les ions terbium.

6. Méthode selon l'une des revendications précédentes, ***caractérisée* en ce qu'**une quantité d'ions lanthanide (III) comprise entre 1 ppm et 10000 ppm est introduite dans l'échantillon, en poids par rapport au poids de l'échantillon avant la mise en contact de l'échantillon avec la solution révélatrice.

7. Méthode selon l'une des revendications précédentes, ***caractérisée* en ce que** la longueur d'onde d'excitation λ_{exc} est comprise entre 200 nm et 600 nm, et **en ce que** la longueur d'onde λₑₘ du signal d'émission est comprise entre 300 nm et 800 nm.

8. Méthode selon l'une des revendications précédentes, ***caractérisée* en ce qu'**un modificateur de signal comprenant un composé cationique est ajouté à l'échantillon avant l'excitation de l'échantillon.

9. Méthode selon l'une des revendications précédentes, ***caractérisée* en ce que** la méthode comprend une étape de purification de l'échantillon avant l'addition de la solution révélatrice comprenant les ions lanthanides (III).

## Patentansprüche

1. Verfahren zum Bestimmen der anionischen Ladungsdichte von mindestens einem in einer Probe vorhandenen Polymer gemäß den folgenden Schritten:
- mindestens ein in der Probe vorhandenes Polymer in Kontakt mit einer Entwicklerlösung, die Lanthanoid (III) -Ionen enthält, bringen und so eine Wechselwirkung zu ermöglichen,
- Anregung der Probe auf eine Anregungswellenlänge λ_{exc} und Detektion durch zeitaufgelöste Photolumineszenz eines Signals, das von den Lanthanid (III) -Ionen stammt, die mit dem mindestens einen Polymer in Wechselwirkung getreten sind mit einer Emissions- Wellenlänge λₑₘ, und
- Bestimmung der anionischen Ladungsdichte des mindestens einen Polymers der Probe unter Verwendung des bei der Emissionswellenlänge λₑₘ detektierten Signals nach dem folgenden Protokoll:
1) es werden verschiedene Probereihen durch sukzessive Auflösung verschiedener Polymer- Stammlösungen mit bekannter Anionizität in Wasser vorbereitet, die Proben jeder Reihe werden dann mit einer Lanthanid- Nachweislösung verdünnt und durch zeitaufgelöste Photolumineszenz (TRPL) analysiert; die Messparameter sowie die Längen der Anregungs- Emissionswellen werden entsprechend der Art des Lanthanids eingestellt,
2) für jede der Reihen werden die Neigungen der Kurven Intensitäten TRPL Signal zu Verdünnungsgrad extrapoliert und wird eine Kalibrierungskurve Neigung zu Anionizität erstellt,
3) Dann wird durch sukzessive Verdünnung einer Probe X mit unbekannter Anionizität eine neue Reihe Proben vorbereitet, nach TRPL- Messung dieser Proben, die Neigung der Kurven Intensitäten TRPL Signal zu Verdünnungsgrad wird extrapoliert
4) nach Korrelation der in 3) extrapolierten Neigung mit der in 2) erarbeiteten Anionizitäts-Kalibrierungsgeraden wird die vorher nicht bekannte Anionizität der Probe X deduziert.

2. Verfahren nach Anspruch 1, ***dadurch gekennzeichnet, dass*** die Probe aus Produktionswasser stammt, das Ergebnis eines Auffangverfahrens für Erdöl oder Gas ist.

3. Verfahren nach einem der vorangehenden Ansprüche, ***dadurch gekennzeichnet, dass*** das mindestens eine in der Probe vorhandene Polymer ein Polymer aus mindestens einem anionischen, wasserlöslichen Monomer und mindestens einem nicht ionischen Monomer (A) und optional mindestens einem kationischen oder zwitterionischen Monomer ist.

4. Verfahren nach einem der vorangehenden Ansprüche, ***dadurch gekennzeichnet, dass*** die Lanthanid- Ionen ausgewählt werden aus Europium-, Terbium-, Samarium- und Dysprosium- Ionen.

5. Verfahren nach einem der vorangehenden Ansprüche, **dadurch gekennzeichnet, dass** es sich bei den Lanthanid- Ionen um Europium- oder Terbium- Ionen handelt.

6. Verfahren nach einem der vorangehenden Ansprüche, ***dadurch gekennzeichnet, dass*** eine Menge von Lanthanid- Ionen (III) zwischen 1 ppm und 10000 ppm in die Probe eingeführt wird, nach Gewicht, bezogen auf das Gewicht der Probe, bevor die Probe mit der Nachweislösung in Kontakt gebracht wird.

7. Verfahren nach einem der vorangehenden Ansprüche, ***dadurch gekennzeichnet, dass*** die Anregungs- Wellenlänge λ_{exc} zwischen 200 nm und 600 nm liegt, und ***dadurch, dass*** die Wellenlänge λₑₘ des Emissionssignals zwischen 300 nm und 800 nm liegt.

8. Verfahren nach einem der vorangehenden Ansprüche, ***dadurch gekennzeichnet, dass*** ein Signalmodifikator, der eine kationische Verbindung enthält der Probe vor der Anregung der Probe zugefügt wird.

9. Verfahren nach einem der vorangehenden Ansprüche, **dadurch gekennzeichnet, dass** das Verfahren einen Schritt der Reinigung der Probe vor der Zugabe der Nachweislösung mit den Lanthanid- Ionen (III) enthält

## Claims

1. Method for determining the anionic charge density of at least one polymer present in a sample, using the following steps:
- bringing the at least one polymer present in the sample into contact with, and enabling its interaction with, a developer solution comprising lanthanide (III) ions,
- exciting the sample at an excitation wavelength of λ_{exc} and detecting, by time-resolved photoluminescence, a signal originating from the lanthanide (III) ions that have interacted with the at least one polymer at an emission wavelength λₑₘ, and
- determining the anionic charge density of the at least one polymer in the sample using the signal detected at the emission wavelength λₑₘ according to the following protocol:
1) different sample series are prepared by successively diluting various stock solutions of polymers of known anionicity with water, the samples from each series are then diluted with a lanthanide developer solution and analyzed by Time-Resolved Fluorescence (TRF); the measurement parameters as well as the emission and excitation wavelengths are adjusted according to the type of lanthanide,
2) for each series, the slopes of the TRF Signal Intensity vs. Dilution Rate curves are extrapolated and a Slope vs. Anionicity calibration curve is developed,
3) a new series of samples is then prepared by successively diluting a sample X of unknown anionicity,
after TRF measurement of these samples, the slope of the TRF Signal Intensity vs Dilution Rate curve is extrapolated,
4) after the extrapolated slope in 3) is correlated with the anionicity calibration line developed in (2), the previously unknown anionicity of the sample X is deduced.

2. Method according to Claim 1 ***characterized in that*** the sample comes from production water from an oil or gas recovery process.

3. Method according to one of the previous claims ***characterized in that*** the at least one polymer present in the sample is a polymer of at least one water-soluble anionic monomer and at least one non-ionic monomer (A) and, optionally, of at least one cationic or zwitterionic monomer.

4. Method according to one of the previous claims ***characterized in that*** the lanthanide ions are chosen from europium, terbium, samarium or dysprosium ions.

5. Method according to one of the previous claims ***characterized in that*** the lanthanide ions are europium or terbium ions.

6. Method according to one of the previous claims ***characterized in that*** an amount of lanthanide (III) ions between 1 ppm and 10,000 ppm is added to the sample by weight based on the weight of the sample before the sample comes into contact with the developer solution.

7. Method according to one of the previous claims ***characterized in that*** the excitation wavelength λ_{exc} lies between 200 nm and 600 nm and the emission signal wavelength λₑₘ lies between 300 nm and 800 nm.

8. Method according to one of the previous claims ***characterized in that*** a signal modifier comprising a cationic compound is added to the sample before the sample is excited.

9. Method according to one of the previous claims ***characterized in that*** the method comprises a sample purification step prior to adding the developer solution comprising the lanthanide (III) ions.
